Europäisches Patentamt

European Patent Office

Office européen des brevets

(11) Veröffentlichungsnummer: **0 006 234**
**A1**

## (12) EUROPÄISCHE PATENTANMELDUNG

(21) Anmeldenummer: 79101967.2

(22) Anmeldetag: 15.06.79

(51) Int. Cl.³: **A 61 K 7/32**

(30) Priorität: 19.06.78 DE 2826758

(43) Veröffentlichungstag der Anmeldung: 09.01.80
Patentblatt 80/1

(84) Benannte Vertragsstaaten: CH GB NL SE

(71) Anmelder: Henkel Kommanditgesellschaft auf Aktien,
Postfach 1100, D-4000 Düsseldorf 1 (DE)

(72) Erfinder: Osberghaus, Rainer, Dr., Flösserstrasse 20,
D-4000 Düsseldorf 13 (DE)

(54) Verwendung einer Kombination von Estern der Citronensäure und/oder Acetylcitronensäure mit Antioxidantien als Deodorantien.

(57) Gegenstand der Erfindung ist die Verwendung einer Kombination von Estern der Citronensäure und/oder Acetylcitronensäure mit aliphatischen oder alicyclischen Alkoholen mit 1-6 C-Atomen im Molekül, insbesondere deren Triestern, mit Antioxidantien in kosmetischen Mitteln als Deodorantien zur Unterdrückung von Körpergeruch.

EP 0 006 234 A1

4000 Düsseldorf, den 15.6.1978
Henkelstraße 67

HENKEL KGaA
ZR-FE/Patente
z-sü

Patentanmeldung

D 5772

"Verwendung einer Kombination von Estern der Citronensäure und/oder Acetylcitronensäure mit Antioxidantien
als Deodorantien"

Es ist bekannt, daß der störende Geruch, der die Perspiration des Menschen begleitet, durch die bakterielle
Zersetzung des zunächst geruchlosen Schweißes verursacht
wird. Um diesem Übelstand zu begegnen, wurden im wesentlichen 2 Wege eingeschlagen, einmal der Einsatz antimikrobieller Verbindungen zur Abtötung der bakteriellen
Hautflora, die die Zersetzung des Schweißes verursacht,
zum anderen der Einsatz von Verbindungen, die die Schweißabsonderung unterbinden. Daneben spielen noch rein sorptiv wirkende sowie geruchsüberdeckende Mittel eine völlig
untergeordnete Rolle. Bei den kosmetischen Mitteln mit
desodorierender Wirkung handelt es sich im Gegensatz zu
den Antiperspirantien durchweg um Mittel mit einem Gehalt
an antimikrobiellen Stoffen. Als solche wurden z.B. Phenolderivate mit und ohne Halogensubstituenten, organische
Quecksilberverbindungen, quartäre Ammoniumverbindungen,
desinfizierend wirkende Abkömmlinge von Aminosäuren vorgeschlagen und zum Teil auch eingesetzt. Wenn bei dem
Einsatz der Deodorantien die Gefahr von Hautreizungen
nicht in so hohem Ausmaß wie bei der Verwendung von Antiperspirantien heraufbeschworen wird, so treten auch bei
der laufenden Benutzung von Antimikrobika enthaltenden
Deodorantien gelegentliche Unverträglichkeiten, Lichtsensibilisierungen und toxische Nebenwirkungen unter-

schiedlicher Stärke auf. Darüber hinaus ist die Mehrzahl dieser Produkte nicht geruchlos, viele besitzen einen leicht phenolischen Geruch. Den aufgezeigten Mängeln konnte durch den Gegenstand des Hauptpatentes, nämlich die Verwendung von Estern der Citronensäure und/oder Acetylcitronensäure mit aliphatischen oder alicyclischen Alkoholen weitgehend abgeholfen werden. Obwohl diese Ester keine schweißhemmenden bzw. antimikrobiellen Mittel im eigentlichen Sinne darstellen, ließen sich mit ihrer Hilfe dennoch sehr gut desodorierende, geruchsneutrale und von Nebenwirkungen weitgehend freie kosmetische Mittel herstellen.

In der deutschen Patentanmeldung P 24 18 338 wird die Verwendung von Estern der Citronensäure und/oder Acetylcitronensäure, insbesondere deren Triestern, mit aliphatischen oder alicyclischen Alkoholen mit 1 - 6 C-Atomen im Molekül in wasserfreien kosmetischen Zubereitungen, in einer Menge von 1 - 25 Gewichtsprozent, bezogen auf die gesamte kosmetische Zubereitung, zur Unterdrückung von Körpergeruch, beschrieben.

/3

Es wurde nun gefunden, daß sich die desodorierende Wirkung der Ester der Citronensäure und/oder Acetylcitronensäure, insbesondere deren Triestern, mit aliphatischen oder alicyclischen Alkoholen mit 1 - 6 Kohlenstoffatomen im Molekül erheblich steigern läßt, wenn man den desodorierenden kosmetischen Mitteln Antioxidantien zusetzt.

Die erfindungsgemäß einzusetzenden Ester der Citronensäure lassen sich in bekannter Weise durch azeotrope Veresterung von Citronensäure mit dem jeweiligen Alkohol herstellen, wie dies z.B. für den Citronensäuretriäthylester in der amerikanischen Patentschrift 2 076 111 (referiert in Chem. Zentralblatt 1937, II., Seite 665) beschrieben ist. Die Herstellung der erfindungsgemäß einzusetzenden Acetylcitronensäureester kann durch Umsetzung der entsprechenden Citronensäureester mit Acetylchlorid, wie dies für den Acetylcitronensäuretriäthylester von Wislicenus in Liebigs Annalen der Chemie Bd. 129, Seite 192, bzw. durch Umsetzung der entsprechenden Citronensäureester mit Essigsäureanhydrid, wie dies gleichfalls für den Acetylcitronensäuretriäthylester in der amerikanischen Patentschrift 2 445 911 (referiert in Chemical Abstracts 1948, Spalte 8818) beschrieben ist, erfolgen.

Als zu veresternde Alkohole kommen z.B. Methanol, Äthanol, Propanol, Isopropanol, Butanol-1, Butanol-2,2-methylpropanol-1, 2-Methylpropanol-2, 2-Methylbutanol-1, 2-Methylbutanol-4, n-Hexylalkohol, Äthylenglykol, Propylenglykol, Trimethylenglykol, Hexamethylenglykol, Glycerin, Erythrit, Sorbit und Cyclohexanol in Frage.

0006234

Unter den erfindungsgemäß einzusetzenden Estern der
Citronensäure als auch der Acetylcitronensäure kommt
sowohl von der desodorierenden Wirkung, als auch von der
anwendungstechnischen Eignung den jeweiligen Triestern
die größte Bedeutung zu und unter diesen wiederum den
Triestern mit aliphatischen einwertigen Alkoholen mit
1 - 6 Kohlenstoffatomen im Molekül.

Als erfindungsgemäß einzusetzende Citronensäureester
bzw. Acetylcitronensäureester sind demnach zum Beispiel
Citronensäuremonomethylester, -monoäthylester, -monopropylester, -monoisopropylester, -mono-n-butylester,
-mono-tert-butylester, -monoamylester, -monohexylester,
-monocyclohexylester, -monoglycerinester, -dimethylester,
-diäthylester, -dipropylester, -diisopropylester,
-dibutylester, -dicyclohexylester, Ester aus 1 Mol
Citronensäure und 2 Mol Propylenglykol, Acetylcitronensäuremonomethylester, -monoäthylester, -monopropylester,
-monobutylester, -monoamylester, -monocyclohexylester,
-dimethylester, -diäthylester, -diisopropylester,
-di-tert.-butylester, -dihexylester, Ester aus 1 Mol
Acetylcitronensäure und 2 Mol Äthylenglykol oder 2 Mol
Hexamethylenglykol, Ester aus 1 Mol Citronensäure beziehungsweise Acetylcitronensäure und 3 Mol Propylenglykol insbesondere jedoch Citronensäuretrimethylester,
-triäthylester, -tripropylester, -triisopropylester,
-tri-n-butylester, -tri-tert.-butylester, -triamylester,
-trihexylester, Acetylcitronensäure-trimethylester,
-triäthylester, -tripropylester, -triisopropylester,
-tri-n-butylester, -tri-tert.-butylester, -triamylester,
-trihexylester zu nennen.

Als erfindungsgemäß einzusetzende Antioxidantien sind alle auf dem pharmazeutischen, kosmetischen und Nahrungsmittel-Sektor gebräuchlichen Antioxidantien geeignet und es sind folgende Produkte zu nennen: Butylhydroxyanisol, Butylhydroxytoluol, Guajakharz, Lecithin, Nordihydroguajaretsäure, Propylgallat, Octylgallat, Dodecylgallat, Tocopherole, Trihydroxybutyrophenon, Ascorbinsäure, Ascorbylpalmitat, Dilaurylthiodipropionat, Distearylthiodipropionat, Monoisopropylcitrat, Thiodipropionsäure und Citraconsäure. Besondere Bedeutung kommt dabei dem Butylhydroxyanisol und Butylhydroxytoluol zu. Für den erfindungsgemäßen Einsatz besonders geeignet hat sich ein Gemisch von 2- und 3-tert. Butyl-4-hydroxyanisol mit 2,6-Di-tert.-butyl-4-methylphenol (Butylhydroxytoluol) im Verhältnis von 1:9 bis 9:1 erwiesen.

Die erfindungsgemäß zu verwendende Kombination von Citronensäureestern bzw. Acetylcitronensäureestern mit Antioxidantien kann in alle üblicherweise für Deodorantien gebräuchliche Zubereitungen eingearbeitet werden wie Puder, Stifte, Roll-on und Sprays, wobei der Deo-Spray das bevorzugte Einsatzgebiet ist. Die Einarbeitung erfolgt in bekannter Weise durch einfaches Vermischen oder Lösen in den anderen Komponenten der Zubereitung wie Lösungsmitteln, Wachsen, Fettsubstanzen, Polyglykolen, Pudergrundstoffen. Die in die erfindungsgemäßen kosmetischen Mittel mit desodorierender Wirkung einzuarbeitenden Mengen an Citronensäureester bzw. Acetylcitronensäureester betragen 0,5 bis 20 Gewichtsprozent, vorzugsweise 2 - 10 Gewichtsprozent, bezogen auf das gesamte Mittel. Die einzuarbeitenden Mengen an Antioxidantien betragen 0,01 bis 5 Gewichtsprozent, vorzugsweise 0,1 bis 3 Gewichtsprozent, bezogen auf das gesamte Mittel.

Es wurde ferner gefunden, daß es nicht erforderlich ist, daß die dem Deodorans zugrundeliegende Zubereitung wasserfrei bzw. wasserarm ist, vielmehr kann

/6

diese erhebliche Mengen Wasser enthalten und in Ausnahmefällen sogar eine wäßrige Lösung darstellen.

Es ist bereits aus der deutschen Offenlegungsschrift 2 358 121 bekannt, die bakterizide Wirkung von Deodorantien durch den Zusatz von Antioxidantien zu steigern. Als bakterizid wirkendes Desodorierungsmittel ist z.B. 2,4,4'-Trichlor-2'-hydroxydiphenyläther, eine allgemein bekannte antimikrobielle Verbindung, genannt. Es war daher überraschend, daß sich die desodorierende Wirkung von Citronensäureestern bzw. Acetylcitronensäureestern, die keine antimikrobiellen Verbindungen darstellen, durch den Zusatz von Antioxidantien erheblich steigern läßt.

Die nachfolgenden Beispiele sollen den Gegenstand der Erfindung näher erläutern, ohne ihn jedoch hierauf zu beschränken.

## B e i s p i e l e

Für eine vergleichende Wirksamkeitsprüfung wurde ein Panel-Test durchgeführt. Dieser wurde so angelegt, daß in einer Testserie jeweils drei Produkte (A, B und C) vergleichend geprüft werden konnten. Zur Vorbereitung der Probanden wurde an 15 Personen je 1 Stück Seife, die keine antimikrobiellen Wirkstoffe enthielt, mit der Anweisung ausgegeben, während der gesamten Testzeit von 3 Wochen außer dieser Seife weder andere Seifen noch Desodorantien bzw. Antiperspirantien noch Parfüms zu benutzen. In der ersten Woche, die als Vorbereitungswoche diente, wurde eine unkontrollierte Wäsche mit der vorgeschriebenen Seife durchgeführt. In der darauffolgenden Woche begann der eigentliche Test: Morgens wurde eine von einer Aufsichtsperson kontrollierte Achselwäsche durchgeführt. Die Testprodukte wurden sodann bei je 5 Probanden nach folgendem Schema unter standardisierten Bedingungen auf die linke bzw. rechte Achsel appliziert:

| linke Achsel | rechte Achsel | |
| --- | --- | --- |
| Produkt A | Produkt B | 5 Probanden |
| Produkt A | Produkt B | 5 Probanden |
| Produkt B | Produkt C | 5 Probanden |

In der dritten Woche wurden die Applikationsseiten gewechselt. Nach Applikation der Testprodukte wurden unter den Achseln der Probanden Achselblätter mittels Gummibändern befestigt, die über einen Zeitraum von 7 Stunden getragen werden mußten. Zur Geruchsbeurteilung wurden die Achselblätter nach Abschluß der Testzeit (7 Stunden) abgenommen, in kodierte, verschließbare und auf 37°C erwärmte Glasbehälter gegeben und die verschraubten Gläser anschließend 15 Minuten bei 37°C gelagert. Die Geruchsbeurteilung des links-rechts-
Unterschiedes der Achselblätter jeder Versuchsperson
(= 1 Prüfobjektpaar) erfolgte durch 3 trainierte
Geruchsprüfer, die ihre Bewertung unabhängig voneinander abgaben. Jeder Prüfer erhielt für jeden Testtag
ein neues Prüfformular und erstellte die Beurteilung
nach folgendem Schema:

stärkerer Geruch  = Note 1
schwächerer Geruch = Note 0
gleicher Geruch   = Note 0,5 für beide Proben.

Der Testleiter stellte sodann die Summe der Benötigungen
der Prüfobjektpaare zusammen:

| | $\leq$ der Bewertung der Prüfobjektpaare | |
|---|---|---|
| A : B | x  Punkte | :  y  Punkte |
| A : C | x' Punkte | :  y' Punkte |
| B : C | x" Punkte | :  y" Punkte |

Durch Addition der Noten, die ein Produkt auf sich
vereinigt, erhält man eine Rangfolge, bei welcher die
jeweils höhere Gesamtnotensumme die schlechtere Wirkung
des Produktes angibt:

| Produkt | Rangfolge |
|---|---|
| A | x + x' Punkte |
| B | y + x" Punkte |
| C | y'+ y" Punkte |

Der Montag als jeweils erster Applikationstag der
Produkte blieb bei der Auswertung in beiden Testwochen unberücksichtigt.

Aus den nachstehend aufgeführten Tabellen ist die
gute Wirksamkeit der erfindungsgemäßen Kombinationen,
die nicht auf einem rein additiven Effekt beruht,
ersichtlich.

In den nachstehenden Versuchen haben die Kurzbezeichnungen
folgende Bedeutung:

BHA = Stellungsisomerengemisch aus 2- und 3-tert.Butyl-
      4-hydroxyanisol.
BHT = 2,6-Di-tert.-butyl-4-methylphenol.
Treibgas 11 = Monofluor-trichlor-methan.
Treibgas 12 = Difluor-dichlor-methan.
Treibgas 114 = 1,2-Tetrafluordichloräthan.

Bei der beschriebenen Bewertung liegt ein signifikanter
Unterschied auf Basis von mindestens 95 % statistischer
Sicherheit bei einer Punktedifferenz von $\geq$ 19 Punkten
vor.

/10

0006234

Patentanmeldung D 5772          - 10 -          HENKEL KGaA
                                                ZR-FE/Patente

Versuch 1

Spray A          3,0 % Citronensäuretriäthylester
                97,0 % Treibgas 11/12   40:60

Spray B          2,8 % Citronensäuretriäthylester
                 0,2 % BHA : BHT = 1:1
                97,0 % Treibgas (wie A)

Spray C          2,8 % Capryl-/Caprinsäuretriglycerid
                 0,2 % BHA : BHT = 1:1
                97,0 % Treibgas (wie A)

| Auswertung | Punkte | Desodorierende Wirkung | Punkte-differenz |
|---|---|---|---|
| Spray A | 120,5 | A > C | 19,5 |
| Spray B | 99,5 | B > A | 21,0 |
|  |  | B > C | 40,5 |
| Spray C | 140,0 |  |  |

Versuch 2

Spray A          5,0 % Acetylcitronensäuretributylester
                 0,05% DL-αTocopherol
                15,0 % Äthanol
                10,0 % Isopropanol
          ad 100 % Treibgas 12

Spray B          5,0 % Acetylcitronensäuretributylester
                15,0 % Äthanol
                10,0 % Isopropanol
          ad 100 % Treibgas (wie A)

/11

0006234

Spray C      0,05 % DL-α-Tocopherol

        1,5 % Isopropylmyristat

      15,0 % Äthanol

      10,0 % Isopropanol

     ad 100 % Treibgas (wie A)

| Auswertung | Punkte | Desodorierende Wirkung | Punkte-differenz |
|---|---|---|---|
| Spray A | 100,5 | A > B | 19,5 |
|  |  | A > C | 39,0 |
| Spray B | 120,0 | B > C | 19,5 |
| Spray C | 139,5 |  |  |

Versuch 3

Spray A      1,5 % Citronensäuretrimethylester

        1,5 % Acetylcitronensäuretriäthylester

        1,5 % BHA : BHT = 1:9

     ad 100    Treibgas 11/12 50:50

Spray B      1,5 % Citronensäuretrimethylester

        1,5 % Acetylcitronensäuretriäthylester

        1,5 % BHA : BHT = 9:1

     ad 100    Treibgas (wie A)

Spray C      1,5 % Citronensäuretrimethylester

        1,5 % Acetylcitronensäuretriäthylester

     ad 100    Treibgas (wie A)

| Auswertung | Punkte | Desodorierende Wirkung | Punkte-differenz |
|---|---|---|---|
| Spray A | 110,0 | A = B | 4,5 |
|  |  | A > C | 34,5 |
| Spray B | 105,5 | B > C | 39,0 |
| Spray C | 144,5 |  |  |

Versuch 4

Spray A     0,2 % 2,4,4'-Trichlor-2'-hydroxydiphenyl-äther

0,8 % Capryl-/Caprinsäuretriglycerid

4,0 % Isopropanol

25,0 % Äthanol

70,0 % Treibgas 11/12 (40:60)

Spray B     3,0 % Citronensäuretriäthylester

0,2 % BHA:BHT = 1:1

4,0 % Isopropanol

22,8 % Äthanol

70,0 % Treibgas (wie A)

Spray C     3,0 % Citronensäuretriäthylester

4,0 % Isopropanol

23,0 % Äthanol

70,0 % Treibgas (wie A)

| Auswertung | Punkte | Desodorierende Wirkung | Punkte-differenz |
|---|---|---|---|
| Spray A | 126,0 | A = C | 8,5 |
| Spray B | 99,5 | B > A | 26,5 |
|  |  | B > C | 35,0 |
| Spray C | 134,5 |  |  |

Versuch 5

Spray A      2,0 % BHT
             5,0 % Capryl-/Caprinsäuretriglycerid
        ad 100    % Treibgas

Spray B      0,05 % BHT
             5,0 % Citronensäuretributylester
        ad 100    % Treibgas

Spray C      0,05 % BHA
             5,0 % Citronensäuretributylester
        ad 100    % Treibgas (wie A)

| Auswertung | Punkte | Desodorierende Wirkung | Punkte-differenz |
|---|---|---|---|
| Spray A | 140,0 |  |  |
| Spray B | 114,5 | B > A | 25,5 |
|  |  | B = C | 9,0 |
| Spray C | 105,5 | C > A | 34,5 |

Versuch 6

Spray A     1,0  % Citronensäuretriäthylester
    1,0  % DL-α-Tocopherol
    38,0 % Äthanol
    60,0 % Treibgas 11/12 40:60

Spray B     1,0  % Citronensäuretriäthylester
    39,0 % Äthanol
    60,0 % Treibgas (wie A)

Spray C     1,0  % DL-α-Tocopherol
    39,0 % Äthanol
    60,0 % Treibgas (wie A)

| Auswertung | Punkte | Desodorierende Wirkung | Punkte-differenz |
|---|---|---|---|
| Spray A | 100,5 | A > B | 19,5 |
|  |  | A > C | 39,5 |
| Spray B | 119,5 | B > C | 20,5 |
| Spray C | 140,0 |  |  |

Die erfindungsgemäße Verwendung der Kombination von Estern der Citronensäure und/oder Acetylcitronensäure mit Antioxidantien kann in kosmetischen Mitteln mit desodorierender Wirkung gemäß nachstehend aufgeführten Rezepturen erfolgen:

Desodorierender Stift

| | | |
|---|---|---|
| 2-Octyldodecanol | 26,5 | Gewichtsteile |
| Cetylstearylalkohol | 3,0 | " |
| Natriumstearat | 8,0 | " |
| Kokosfettsäuremonoäthanolamid | 3,0 | " |
| Paraffinöl | 2,0 | " |
| Propylenglykol | 2,0 | " |
| Äthanol | 50,0 | " |
| Citronensäuretributylester | 5,0 | " |
| BHA + BHT 1:1 | 0,5 | " |

Desodorierender Puder

| | | |
|---|---|---|
| Reisstärke Derivat NAL R-5 | 10,0 | Gewichtsteile |
| Magnesiumcarbonat | 2,C | " |
| Zinkoxid | 2,0 | " |
| Talkum extra fein | 79,5 | " |
| Acetylcitronensäuretri-propylester | 6,0 | " |
| BHA + BHT 1:1 | 0,5 | " |

Desodorierender Spray

| | | |
|---|---|---|
| Citronensäuretriäthylester | 6,0 | Gewichtsteile |
| BHA + BHT 1:1 | 0,5 | " |
| Äthanol | 29,3 | " |
| Isopropanol | 3,0 | " |
| Propylenglykol | 1,2 | " |
| Treibgas 12/114 60:40 | 60,0 | " |

Desodorierender Spray

| | | |
|---|---|---|
| Acetylcitronensäuretriäthylester | 4,7 | Gewichtsteile |
| α-Tocopherol | 0,3 | " |
| Äthanol | 10,0 | " |
| Isopropanol | 18,0 | " |
| Isopropylmyristat | 2,0 | " |
| Treibgas 12/114 60:40 | 65,0 | " |

Desodorierender Spray

| | | |
|---|---|---|
| Citronensäuretributylester | 5,0 | Gewichtsteile |
| BHA | 0,5 | " |
| Capryl/Caprinsäuretriglycerid | 4,0 | " |
| Treibgas (Frigen) 12/114 60:40 | 90,5 | " |

Desodorierender Spray

| | | |
|---|---|---|
| Citronensäuretrimethylester | 10,0 | Gewichtsteile |
| BHT | 1,0 | " |
| Propylenglykol | 1,5 | " |
| Isopropylstearat | 1,5 | " |
| Treibgas 12/114 60:40 | 86,0 | " |

Desodorierender Spray

| | | |
|---|---|---|
| Citronensäuretrihexylester | 7,5 | Gewichtsteile |
| BHA + BHT 1:1 | 0,5 | " |
| Propylenglykol | 2,0 | " |
| Isopropylmyristat | 2,0 | " |
| Äthanol | 11,0 | " |
| Treibgas 11/12 50:50 | 77,0 | " |

0006234

### Desodorierender Spray

| | | |
|---|---|---|
| Acetylcitronensäuretricyclo-hexylester | 8,0 | Gewichtsteile |
| α-Tocopherol | 0,5 | " |
| Äthanol | 27,0 | " |
| Isopropylmyristat | 3,0 | " |
| Treibgas | 61,5 | " |

### Deo-Roll-on

| | | |
|---|---|---|
| Äthanol | 45,0 | Gewichtsteile |
| Citronensäuretriäthylester | 1,0 | " |
| BHA + BHT 1:4 | 0,2 | " |
| Acrylsäurepolymerisat der Fa. Goodrich Chem. Corp. Carbopol 940(R) | 0,2 | " |
| Triäthanolamin | 0,1 | " |
| Wasser | 49,5 | " |
| Nonylphenolpolyglykoläther der Elektrochem. Fabr. Kempen Merpoxen NO(R) | 4,0 | " |

### Deodorant-Creme

| | | |
|---|---|---|
| Gemisch von Mono- und Diglyceriden der Palmitin- und Stearinsäure Cutina MD(R) Dehydag | 18,0 | Gewichtsteile |
| Cetyl/Stearylalkohol + ca. 12 Mol Äthylenoxid Eumulgin B 1(R) Dehydag | 4,0 | " |
| Ölsäuredecylester | 10,0 | " |
| Citronensäuretriäthylester | 5,0 | " |
| BHA + BHT 1:9 | 0,5 | " |
| Wasser | 61,4 | " |
| p-Hydroxybenzoesäuremethylester | 0,1 | " |
| Parfümöl | 1,0 | " |

Deodorans für Pumpzerstäuber

| | | |
|---|---|---|
| Äthanol | 81,0 | Gewichtsteile |
| Isopropanol | 4,0 | " |
| Citronensäuretriäthylester | 1,0 | " |
| BHA + BHT 9:1 | 0,3 | " |
| Parfüm | 1,0 | " |
| Wasser | 12,7 | " |

Deodorans für Pumpzerstäuber

| | | |
|---|---|---|
| Äthanol | 50,0 | Gewichtsteile |
| Citronensäuretriäthylester | 1,5 | " |
| BHA + BHT 5:1 | 0,1 | " |
| Parfüm | 1,0 | " |
| Wasser | 47,4 | " |

Patentanmeldung D 5772          1          HENKEL KGaA
ZR-FE/Patente

"Verwendung einer Kombination von Estern der Citronensäure und/oder Acetylcitronensäure mit Antioxidantien
als Deodorantien"

Patentansprüche:

1. Verwendung von Estern der Citronensäure und/oder
   Acetylcitronensäure, insbesondere deren Triestern,
   mit aliphatischen oder alicyclischen Alkoholen mit
   1 - 6 Kohlenstoffatomen im Molekül in wasserfreien
   kosmetischen Zubereitungen in einer Menge von 1 - 25
   Gewichtsprozent, bezogen auf die gesamte kosmetische
   Zubereitung, zur Unterdrückung von Körpergeruch
   dadurch gekennzeichnet, daß die Ester der Citronensäure und/oder Acetylcitronensäure in einer Menge von
   0,5 - 20 Gewichtsprozent in Kombination mit Antioxidantien in einer Menge von 0,01 bis 5 Gewichtsprozent, beide jeweils bezogen auf die gesamte Zubereitung, verwendet werden.

2. Verwendung von Estern der Citronensäure und/oder
   Acetylcitronensäure in Kombination mit Antioxidantien
   nach Anspruch 1, dadurch gekennzeichnet, daß die
   Ester der Citronensäure und/oder Acetylcitronensäure
   in einer Menge von 1 - 10 Gewichtsprozent und die
   Antioxidantien in einer Menge von 0,1 - 3 Gewichtsprozent, jeweils bezogen auf die gesamte Zubereitung,
   verwendet werden.

/2

0006234

Patentanmeldung D 5772          2          HENKEL KGaA
                                           ZR-FE/Patente

3. Verwendung von Estern der Citronensäure und/oder
   Acetylcitronensäure in Kombination mit Antioxidantien
   nach Anspruch 1 und 2, dadurch gekennzeichnet, daß
   die Verwendung sowohl in wasserfreien wie auch
   wasserhaltigen kosmetischen Zubereitungen erfolgt.

4. Verwendung von Estern der Citronensäure und/oder
   Acetylcitronensäure in Kombination mit Antioxidantien
   nach Anspruch 1 - 3, dadurch gekennzeichnet, daß
   als Antioxidantien ein Gemisch von 2- und 3-tert.-
   Butyl-4-hydroxyanisol mit 2,6-Di-tert.-butyl-4-
   methylphenol im Verhältnis von 1:9 bis 9:1 eingesetzt wird.

0006234

| | | |
|---|---|---|
| ))) | Europäisches Patentamt | **EUROPÄISCHER RECHERCHENBERICHT** |

Nummer der Anmeldung

**EP 79 10 1967**

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 456 639 (H. SCHWARZKOPF) <br> * Patentansprüche; Seite 3, Abschnitt 2 - Seite 4, Abschnitt 1; Seite 5, Abschnitt 2; Beispiele 4,7,12 * <br><br> -- | 1-4 |
| A | FR - A - 2 362 210 (UNILEVER) <br> * Seite 1, Zeile 3 - Seite 3, Ende; Patentansprüche * <br><br> -- | 1 |
| DA | DE - A - 2 418 338 (HENKEL) <br> * Patentansprüche * <br><br> -- | 1 |
| A | FR - A - 2 192 799 (GILLETTE) <br> * Patentansprüche * <br><br> -- | 1 |
| AD | DE - A - 2 358 121 (BEECHAM) <br> * Patentansprüche; Seite 2, Abschnitt 6 - Seite 3, Abschnitt 1 * <br><br> -- | 1 |
| A | FR - A - 2 160 898 (THE MENNEN CY.) <br> * Patentansprüche * <br><br> -- | 1 |
| A | FR - A - 2 099 582 (L'OREAL) <br> * Patentansprüche, Seite 2, Zeilen 10-12; Beispiele * <br><br> ---- | 1 |

**KLASSIFIKATION DER ANMELDUNG (Int.Cl.²)**

A 61 K 7/32

**RECHERCHIERTE SACHGEBIETE (Int. Cl.²)**

A 61 K 7/32

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 07-09-1979 | VANHECKE |

EPA form 1503.1 06.78